# EUROPEAN PATENT APPLICATION

(11) **EP 4 667 465 A1**
(43) Date of publication of application: **24.12.2025**
(21) Application number: 24756269.7
(22) Date of filing: 07.02.2024
(51) Int. Cl.: C07D 451/06, C07D 471/04, C07D 495/04, C07D 401/06, C07D 401/02, A61K 31/706, A61K 31/454

(54) **SALT OF BENZO-NITROGEN HETEROAROMATIC RING DERIVATIVE, CRYSTALLINE FORM, AND PHARMACEUTICAL USE THEREOF**

(30) Priority: 16.02.2023 CN 202310123898
(71) Applicant: Haisco Pharmaceutical Group Co., Ltd., Shannan, Tibet 856000 (CN)
(72) Inventor: GONG, Zheng, Lhoka, Tibet 856099 (CN); WANG, Chengtao, Lhoka, Tibet 856099 (CN); JIANG, Qi, Lhoka, Tibet 856099 (CN); FAN, Jiang, Lhoka, Tibet 856099 (CN); DOU, Ying, Lhoka, Tibet 856099 (CN)
(74) Representative: AWA Denmark A/S
(86) International application number: PCT/CN2024/076765
(87) International publication number: WO 2024/169896

(57) **Abstract**

The present invention relates to a pharmaceutically acceptable succinic acid salt of a compound as shown in formula (I) or a stereoisomer of the compound, a crystalline form, a preparation method, a pharmaceutical composition thereof, and a use in preparation of a drug for treating diseases related to the activity or expression level of complement factor B.

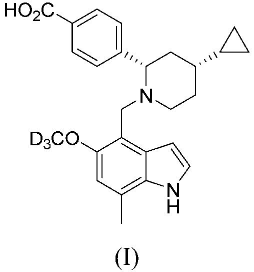

## Description

### Technical Field

The present invention relates to the field of medicine, and in particular to a pharmaceutically acceptable succinic acid salt of a compound represented by formula (I) or a stereoisomer of the compound, a crystalline form and a preparation method therefor, and also to a pharmaceutical composition thereof and a use in the preparation of a drug for treating diseases related to the activity or expression level of complement factor B.

### Background Art

Complement factor B is a component of the complement alternative pathway and participates in the body's specific and non-specific immune mechanisms. It contains a serine protease (SP) domain. When activated, it will provide catalytic activity of C3 and C5 convertases of the alternative pathway. Complement factor B is a key enzyme in the activation process of the complement alternative pathway and can be used as a suitable target to inhibit the complement activation pathway.

Patent PCT/CN 2022/113216 discloses a compound represented by formula (I), which has good inhibitory activity against complement factor B and has good inhibition rate of C3a level *in vivo.* When the compound is used as a therapeutic agent for treating humans, the administration of a therapeutically effective dose becomes problematic and may lead to toxic side effects or ineffective therapy. Therefore, it is very important to choose a salt form that is stable and reproducibly manufacturable and possesses physicochemical properties favorable for its use as a therapeutic agent.

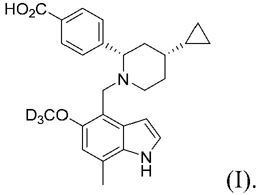

### Summary of the Invention

The objective of the present invention is to provide a pharmaceutically acceptable succinic acid salt of a compound represented by formula (I) or a stereoisomer of the compound, a crystalline form, a preparation method, and a use in the preparation of a drug for treating diseases related to the activity or expression level of complement factor B.

The advantages of the salt and crystalline form of the compound of the present invention include, but are not limited to, ease of processing and crystallization, convenient handling, ease of purification and industrial-scale production, good flowability, ease of micronization, high solubility, low hygroscopicity, favorable pharmacokinetic properties, good stability, and suitability for the preparation of pharmaceutical formulations.

The present invention provides a succinic acid salt and a crystalline form of a compound represented by formula (I),

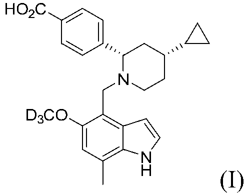

In some embodiments, the pharmaceutically acceptable salt is selected from succinic acid salts.

In some embodiments, the molar ratio of the compound represented by formula (I) to succinic acid is about 1 : 0.5 to 1 : 2.5.

In some embodiments, the molar ratio of the compound represented by formula (I) to succinic acid is about 1 : 0.75.

The present invention relates to a crystalline form 1 of a succinic acid salt of the compound represented by formula (I) above, which has an X-ray powder diffraction pattern with characteristic diffraction peaks at the following 2θ positions, determined using Cu-Kα radiation: 7.77° ± 0.2°, 8.17° ± 0.2°, 14.61° ± 0.2°, 17.06° ± 0.2°, and 20.00° ± 0.2°.

In some embodiments, the preceding crystalline form 1 of the succinic acid salt of the compound represented by formula (I) has an X-ray powder diffraction pattern with characteristic diffraction peaks at the following 20 positions, determined using Cu-Kα radiation: 7.77° ± 0.2°, 8.17° ± 0.2°, 9.27° ± 0.2°, 12.76° ± 0.2°, 14.61° ± 0.2°, 17.06° ± 0.2°, 20.00° ± 0.2°, and 22.48° ± 0.2°.

In some embodiments, the preceding crystalline form 1 of the succinic acid salt of the compound represented by formula (I) has an X-ray powder diffraction pattern with characteristic diffraction peaks at the following 2θ positions, determined using Cu-Kα radiation: 7.34° ± 0.2°, 7.77° ± 0.2°, 8.17° ± 0.2°, 9.27° ± 0.2°, 12.50° ± 0.2°, 12.76° ± 0.2°, 13.69° ± 0.2°, 14.61° ± 0.2°, 17.06° ± 0.2°, 19.76° ± 0.2°, 20.00° ± 0.2°, 21.52° ± 0.2°, 22.01° ± 0.2°, 22.48° ± 0.2°, 22.56° ± 0.2°, and 24.53° ± 0.2°.

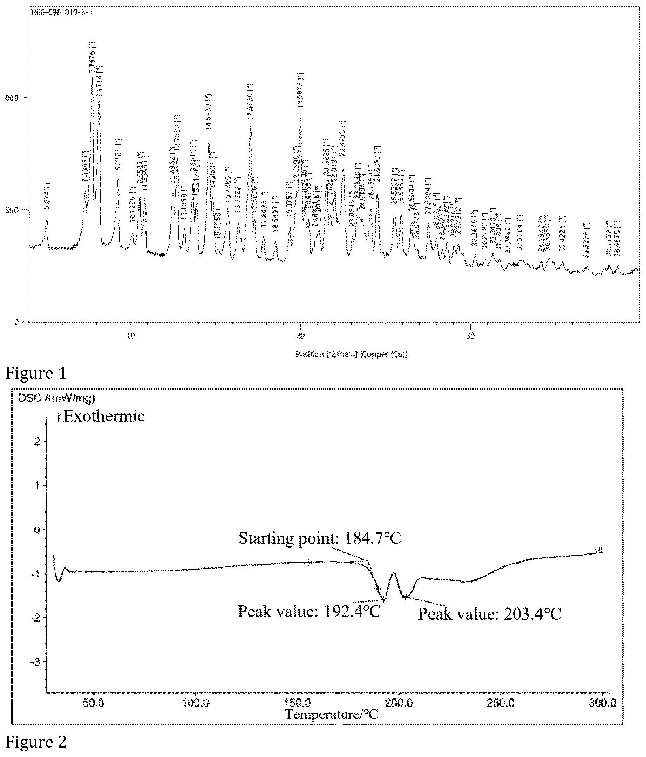

In some embodiments, the preceding crystalline form 1 of the succinic acid salt of the compound represented by formula (I) has an X-ray powder diffraction pattern as shown in Figure 1, determined using Cu-Kα radiation.

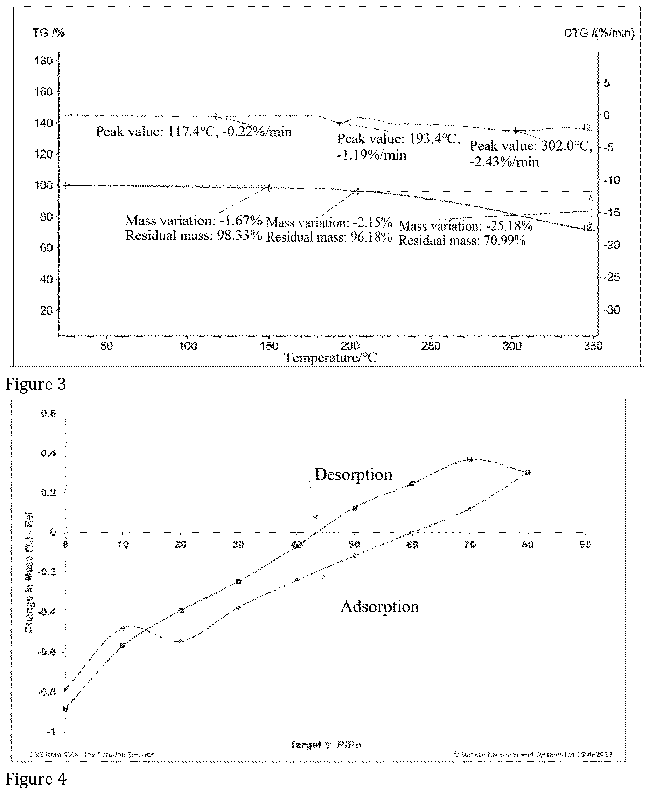

In some embodiments, the preceding crystalline form 1 of the succinic acid salt of the compound represented by formula (I) has a differential scanning calorimetry (DSC) curve showing the peak temperatures of the crystalline form 1 being 192.4°C and 203.4°C, respectively; a thermogravimetric analysis (TGA) curve showing a weight loss of about 1.67% before the temperature reaches 150°C; and a sorption isotherm curve showing a hygroscopic weight gain of about 1.15% at 80% RH; and the differential scanning calorimetry curve, the thermogravimetric analysis curve and the sorption isotherm curve thereof are as shown in Figures 2-4.

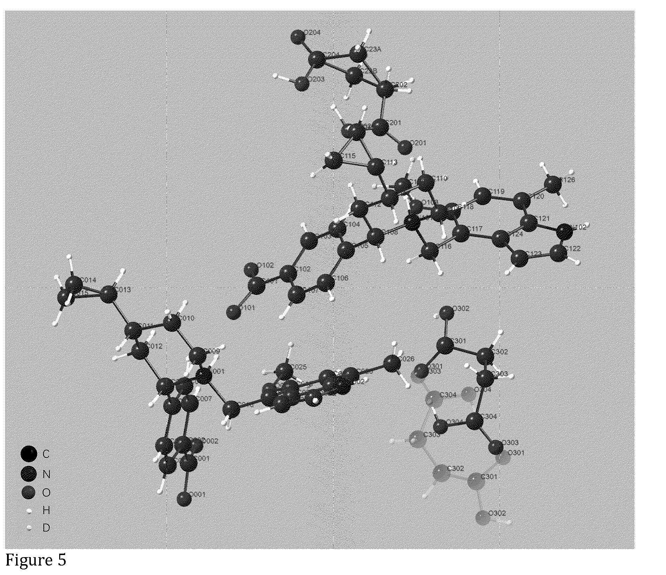

In some embodiments, the crystal structure of the aforementioned compound represented by formula (I) is successfully determined by means of the MicroED method. The unit cell parameters are shown in Table 1. The crystal structure belongs to the orthorhombic crystal system with a space group C2221 (No. 20), the lattice constants are a = 12.66(3) Å, b = 23.79(3) Å, c = 34.40(6) Å, α = 90°, β = 90° and γ = 90°, the unit cell volume V = 10364(36) Å3, and the Z' of the system is 2. In some embodiments, an asymmetric unit of the crystalline form 1 consists of 2 molecules of the compound represented by formula (I) and 1.5 molecules of succinic acid. In some embodiments, an asymmetric unit in the unit cell is as shown in Figure 5.

**Table 1 Unit cell parameters of crystalline form 1 of succinic acid salt of compound represented by formula (I):**

| | |
|---|---|
| Empirical formula | C₂₉H_{31.50}D₃N₂O₆ |
| Chemical formula | C₂₆H₂₇D₃N₂O₃ · 0.75 C₄H₆O₄ |
| Molecular weight | 510.11 g·mol⁻¹ |
| Temperature | 98(2) K |
| Wavelength | 0.02508 Å |
| Crystal system, space group | Orthorhombic, C222₁ (No. 20) |
| Unit cell parameters | a = 12.66(3) Å |
| | b = 23.79(3) Å |
| | c = 34.40(6) Å |
| | α = 90° |
| | β = 90° |
| | γ = 90° |
| Volume | 10364(36) Å³ |
| Z' | 2 |

The present invention further relates to a method for preparing a crystalline form 1 of a succinic acid salt of a compound represented by formula (I), wherein the method comprises: (1) adding a solvent (i) to a compound represented by formula (I), and stirring same at room temperature or under heating; and (2) adding an aqueous succinic acid solution, and stirring and crystallizing same, followed by drying to obtain the target crystalline form 1. In some embodiments, the solvent used is selected from one of a C₁₋₆ halogenated alkane solvent, a C₂₋₆ ester solvent, a C₂₋₆ nitrile solvent, a C₂₋₆ ether solvent, a C₁₋₆ alcohol solvent or water or a mixed solvent of two or more thereof at any ratio, preferably one or more of dichloromethane, 1,2-dichloroethane, acetonitrile, ethyl acetate, methanol, ethanol, isopropanol, propanol, diethyl ether, tetrahydrofuran or water.

In some embodiments, in the aforementioned preparation method of the crystalline form 1, the solvent (i) is selected from a mixed solvent of ethanol and water or ethanol; and the volume (ml) of the solvent (i) is 1 to 100 times the weight (g) of the compound, preferably 1 to 50 times, and more preferably 2 to 8 times.

In one aspect, the present invention further provides a pharmaceutical composition, wherein the pharmaceutical composition comprises a therapeutically effective amount of the aforementioned succinic acid salt and crystalline form of the compound represented by formula (I), and a pharmaceutically acceptable carrier or excipient.

The term "effective amount" or "therapeutically effective amount" in the present application refers to a sufficient amount of the compound disclosed in the present application that is administered to ameliorate, to some extent, one or more symptoms of a disease or condition being treated (e.g., a kidney disease). In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease.

In another aspect, the present invention further provides a use of the aforementioned pharmaceutically acceptable salt of the compound represented by formula (I), or the succinic acid salt and crystalline form of the compound represented by formula (I), or the aforementioned pharmaceutical composition in the preparation of a drug for treating diseases related to kidney diseases.

The present invention relates to a kit, wherein the kit may comprise a composition in the form of a single dose or multiple doses, and the kit comprises the pharmaceutically acceptable salt or cocrystal of the compound according to the present invention, with the amount of the pharmaceutically acceptable salt or cocrystal of the compound according to the present invention being identical to the amount thereof in the aforementioned pharmaceutical composition.

The crystalline form of the compound represented by formula (I) according to the present invention has excellent physical properties including, but not limited to, excellent solubility, dissolution rate, and light resistance, low hygroscopicity, high-temperature resistance and high-moisture resistance. For example, the crystalline form of the present invention can significantly reduce the filtration time, shorten the production cycle, and save costs when being used to prepare a formulation. Moreover, the crystalline form of the present invention also has good photostability, thermal stability and humidity stability, which can ensure the reliability of the crystalline form during storage and transportation, thereby ensuring the safety of the formulation. In addition, the crystalline form does not require special packaging against light, temperature and humidity, thereby reducing the costs. The crystalline form will not degrade due to the effects of light, high temperature and high humidity, thereby improving the safety and long-term efficacy of the formulation. Patients taking the crystalline form do not need to worry about photosensitivity reactions caused by the formulation due to exposure to sunlight.

The crystalline form of the compound represented by formula (I) according to the present invention has good chemical and physical stability, is easy to prepare and is more suitable for use in formulation preparation. The crystalline form of the present invention has the advantages of good flowability, good compressibility, high bulk density, low hygroscopicity and uniform particle size distribution.

The crystalline form of the compound represented by formula (I) of the present invention is suitable and convenient for large-scale preparation. A formulation prepared with the crystalline form can achieve reduced irritation and improved absorption, thereby addressing issues related to metabolic rates, significantly reducing toxicity, improving safety, and effectively ensuring the quality and efficacy of the formulation.

It can be understood that the expression "preferably, ... has an X-ray powder diffraction pattern with characteristic diffraction peaks at the following 20 positions" or "more preferably, ... has an X-ray powder diffraction pattern with characteristic diffraction peaks at the following 2θ positions" and other similar expressions of the present invention mean that in addition to comprising characteristic diffraction peaks at 20 positions described above, the X-ray powder diffraction pattern further comprises characteristic diffraction peaks at "the following 2θ positions".

It can be understood that the numerical values described and claimed in the present invention are approximate values. Changes in values may be attributed to device calibration, device errors, crystal purity, crystal size, sample size and other factors.

The structure of the crystalline form of the present invention can be analyzed by using various analytical techniques known to those skilled in the art, including but not limited to, X-ray powder diffraction (XRD), ion chromatography (IC), differential scanning calorimetry (DSC) and/or thermogravimetric analysis (TGA), also known as thermogravimetry (TG).

It can be understood that the crystalline forms of the present invention are not limited to the characteristic patterns such as XRD, DSC and TGA which are completely identical to those described in the drawings disclosed in the present invention, and any crystalline form having a characteristic pattern which is substantially or essentially the same as those described in the drawings falls within the scope of the present invention.

It can be understood that, as is well known in the field of differential scanning calorimetry (DSC), a melting peak height of a DSC curve depends on many factors related to sample preparation and geometric shapes of instruments, and a peak position is relatively insensitive to experiment details. Therefore, in some embodiments, the DSC patterns having the characteristic peak positions of the crystallized compounds of the present invention have substantially the same properties as the DSC patterns provided in the drawings of the present invention, with an error tolerance of ± 3°C.

Unless otherwise specified, the technical and scientific terms used herein have the same meaning as commonly understood by those skilled in the art to which the present invention pertains. In case of conflict, the definitions provided in the present application will control. When an amount, concentration or other value or parameter is expressed in the form of a range, a preferred range, or a preferred upper numerical limit and a preferred lower numerical limit, it should be understood as specifically disclosing any range formed by combining any upper limit or preferred value with any lower limit or preferred value, regardless of whether the range is specifically enumerated. Unless otherwise specified, all numerical ranges listed herein are intended to include the endpoints of the range and all integers and fractions (decimals) within the range.

Unless stated to the contrary, the terms used in the description and the claims have the following meanings.

The term "optional" or "optionally" in the present invention refers to that the events or circumstances subsequently described may but not necessarily occur, and the description includes the occasions where the events or circumstances occur or do not occur.

The terms "about" and "approximately" in the present invention, when used in conjunction with numerical variables, typically mean that a numerical value of the variable and all numerical values of the variable are within the experimental error (e.g., within a 95% confidence interval of the mean), or within ± 10% of a specified value, or within a broader range.

Unless otherwise specified, all percentages, parts, etc., herein are by weight.

The term "amorphous" in the present invention refers to any solid substance that is not ordered in three dimensions. In some cases, an amorphous solid can be characterized by known techniques, including XRPD crystal diffraction analysis, differential scanning calorimetry (DSC), solid-state nuclear magnetic resonance (ssNMR) spectroscopy analysis or a combination thereof. As explained below, amorphous solids produce an XRPD pattern with no distinct characteristic diffraction peaks. Other characterization methods, such as differential scanning calorimetry (DSC), thermogravimetric analysis (TGA) and dynamic vapor sorption (DVS), further aid in identifying crystalline forms and in determining stability and solvent/water content.

The term "crystalline form" or "crystal" in the present invention refers to any solid substance that exhibits a three-dimensional order, and in contrast to an amorphous solid substance, produces a characteristic XRPD pattern with well-defined peaks.

The term "pharmaceutical composition" in the present invention represents a mixture of one or more compounds described herein or physiologically/pharmaceutically acceptable salts thereof and other components, wherein the other components comprise physiologically/pharmaceutically acceptable carriers and excipients.

The term "carrier" in the present invention refers to a carrier or diluent that does not cause significant irritation to the organism and does not eliminate the biological activity and characteristics of the administered compound.

The term "excipient" in the present invention refers to an inert substance incorporated into a pharmaceutical composition, depending on the administration of a compound. Examples of excipients include, but are not limited to, calcium carbonate, calcium phosphate, various sugars and various types of starch, cellulose derivatives (including microcrystalline cellulose), gelatin, vegetable oil, polyethylene glycols, diluents, granulating agents, lubricants, binders, disintegrants, etc.

The "ether solvent" in the present invention refers to a chain compound or a cyclic compound containing an ether bond -O- and having 1-10 carbon atoms, and the specific examples thereof include, but are not limited to: tetrahydrofuran, diethyl ether, propylene glycol methyl ether, methyl tert-butyl ether, isopropyl ether or 1,4-dioxane.

The "alcohol solvent" in the present invention refers to a group derived from "C₁₋₆ alkyl" on which one or more hydrogen atoms are substituted with one or more "hydroxyl groups", wherein the "hydroxyl group" and "C₁₋₆ alkyl" are as defined above, and the specific examples thereof include, but are not limited to: methanol, ethanol, isopropanol, n-propanol, isopentanol or trifluoroethanol.

The "ester solvent" in the present invention refers to a combination of a lower organic acid containing 1-4 carbon atoms and a lower alcohol containing 1-6 carbon atoms, and the specific examples thereof include, but are not limited to: ethyl acetate, isopropyl acetate or butyl acetate.

The "ketone solvent" in the present invention refers to a compound in which a carbonyl group (-C(O)-) is connected to two hydrocarbon groups. According to different hydrocarbon groups in molecules, ketones can be classified into aliphatic ketones, alicyclic ketones, aromatic ketones, saturated ketones and unsaturated ketones, and the specific examples thereof include, but are not limited to: acetone, acetophenone and 4-methyl-2-pentanone.

The "nitrile solvent" in the present invention refers to a group derived from "C₁₋₆ alkyl" on which one or more hydrogen atoms are substituted with one or more "cyano groups", wherein the "cyano group" and "C₁₋₆ alkyl" are as defined above, and the specific examples thereof include, but are not limited to: acetonitrile or propionitrile.

The "halogenated hydrocarbon solvent" in the present invention refers to a group derived from "C₁₋₆ alkyl" on which one or more hydrogen atoms are substituted with one or more "halogen atoms", wherein the "halogen atom" and "C₁₋₆ alkyl" are as defined above, and the specific examples thereof include, but are not limited to: dichloromethane, 1,2-dichloroethane, chloroform or carbon tetrachloride.

The "room temperature" in the present invention generally refers to 4°C - 30°C, preferably 20°C ± 5°C.

The drying temperature in the present invention is generally 20°C - 100°C, preferably 25°C - 70°C. The drying may be drying under ambient pressure or drying under reduced pressure (vacuum drying). Preferably, the drying is carried out under reduced pressure.

The term "X-ray powder diffraction pattern (XRPD pattern)" in the present invention refers to an experimentally observed diffraction pattern or parameters, data or values derived therefrom. An XRPD pattern is typically characterized by peak positions (abscissa) and/or peak intensities (ordinate).

The term "20 or 20 angle" in the present invention refers to a diffraction angle, where θ is the Bragg angle. The 20 represents the position of a peak expressed in degrees (°) and set based on the X-ray diffraction experiment and is typically the unit of the abscissa in the diffraction pattern. If the reflection beam is diffracted when the incident beam forms an angle θ with a lattice plane, the experimental setup requires that the reflection beam be recorded at an angle 20. It should be understood that a particular 20 value for a particular crystalline form referred to herein is intended to mean the 20 value (expressed in degrees) measured using the X-ray diffraction experimental conditions described herein, and the error range of the 20 is ± 0.3, and may be ± 0.3, ± 0.2 or ± 0.1.

The expression "substantially the same" in the present invention means that representative peak positions and intensity variations are considered. For example, those skilled in the art will appreciate that peak positions (20) may have some variations, typically a variation of up to 0.1-0.2°, and the instruments used to measure the diffraction can also cause some variations. In addition, those skilled in the art will appreciate that relative peak intensity may vary due to instrument-to-instrument differences, degree of crystallinity, preferential orientation, prepared sample surfaces, and other factors known to those skilled in the art and should be considered as qualitative measurements only.

The term "differential scanning calorimetry or DSC" in the present invention refers to measuring the temperature difference and the heat flow difference between a sample and a reference substance when the sample is heated or kept at a constant temperature to characterize all physical and chemical changes related to the thermal effect, thereby obtaining the phase transition information of the sample.

According to the description of the hygroscopicity characteristics and the definition of hygroscopic weight gain in the Chinese Pharmacopoeia 2020 Edition, Volume IV, General Chapter 9103: Guidelines for Hygroscopicity Trials for Pharmaceuticals,
deliquescence: absorbing enough moisture to form a liquid;
extremely hygroscopic: a hygroscopic weight gain of not less than 15%;
hygroscopic: a hygroscopic weight gain of less than 15% but no less than 2%;
slightly hygroscopic: a hygroscopic weight gain of less than 2% but no less than 0.2%; and
non-hygroscopic or practically non-hygroscopic: a hygroscopic weight gain of less than 0.2%.

The crystallization method in the present invention includes, but is not limited to, crystal slurrying crystallization, volatilization crystallization, anti-solvent crystallization, cooling crystallization, and vapor-induced crystallization or diffusion crystallization.

The crystalline form disclosed in the present invention can be prepared by the following common methods for preparing crystalline forms:
1. a volatilization experiment, in which a clear solution of a sample is exposed to an atmosphere at various temperatures until the solvent is volatilized and removed;
2. a crystal slurrying experiment, in which a supersaturated solution of a sample (containing an undissolved solid) is stirred in different solvent systems at a certain temperature;
3. an anti-solvent experiment, in which a sample is dissolved in a good solvent, and an anti-solvent is added to precipitate a solid, followed by brief stirring and immediate filtration;
4. a cooling crystallization experiment, in which a certain amount of samples is dissolved in a corresponding solvent at a high temperature, and the mixture is directly stirred at room temperature or a low temperature for crystallization;
5. a polymer template experiment, in which various polymer materials are added to clear solutions of a sample, and the resulting solution is exposed to an atmosphere at room temperature until the solvent is volatilized and removed;
6. a thermal method experiment, in which a sample is treated according to a certain thermal method under crystallization conditions and cooled to room temperature; and
7. a water vapor diffusion experiment, in which a sample is left in a certain humidity environment at room temperature.

### Brief Description of the Drawings

Figure 1 shows the X-ray powder diffraction pattern of the crystalline form 1 of a succinic acid salt of compound 1.
Figure 2 shows the differential scanning calorimetry curve pattern of the crystalline form 1 of a succinic acid salt of compound 1.
Figure 3 shows the thermogravimetric analysis pattern of the crystalline form 1 of a succinic acid salt of compound 1.
Figure 4 shows the sorption isotherm curve pattern of the crystalline form 1 of a succinic acid salt of compound 1.
Figure 5 shows a single-crystal structural diagram of the crystalline form 1 of a succinic acid salt of compound 1.

### Detailed Description of Embodiments

The structures of the compounds are determined by nuclear magnetic resonance (NMR) or (and) mass spectrometry (MS). The NMR shift (δ) is given in the unit of 10⁻⁶ (ppm). NMR is determined with a WNMR-I 400 nuclear magnetic resonance instrument; the solvent for determination is deuterated dimethyl sulfoxide (DMSO-d₆), deuterated chloroform (CDCl₃) and deuterated methanol (CD₃OD); and the internal standard is tetramethylsilane (TMS).

XRD is determined with a Malvern Panalytical X-ray powder diffractometer. The 20 scan angle is 4-40° 20, and the scan step size is 0.01°. For the test of a sample, the tube voltage and current are 40 kV and 40 mA, respectively, and the sample pan is a zero-background sample pan.

TGA test conditions: the model of the thermogravimetric analyzer is NETZSCH (DSC 214). 1-10 mg of a sample is placed in a balanced sample pan and automatically weighed in a TGA furnace. The sample is heated to the final temperature at a rate of 10°C/min with a nitrogen purge rate of 30 mL/min at the sample.

DSC test conditions: the model of the differential scanning calorimeter is NETZSCH (NETZSCH TG 209 F3). 0.5-5 mg of a sample is accurately weighed and placed in a standard pan or a perforated aluminum crucible sample pan and heated to the final temperature at a rate of 10°C/min with a nitrogen purge rate of 40 mL/min in the furnace.

The known starting materials of the present invention can be synthesized using or according to methods known in the art.

Unless otherwise specified in the examples, a solution refers to an aqueous solution.

The implementation process and beneficial effects of the present invention are described in detail below through specific examples, which are intended to help readers better understand the essence and characteristics of the present invention, and are not intended to limit the scope of implementation of the present invention.

### Example 1: preparation of compound 1

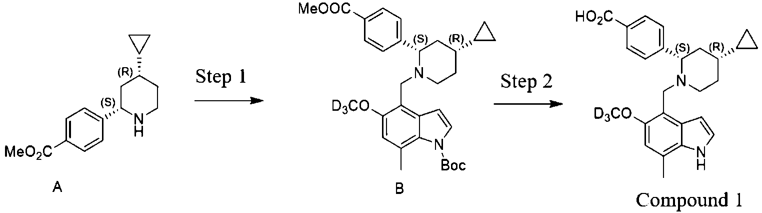

### Step 1: Synthesis of intermediate B

### tert-Butyl 4-(((2S,4R)-4-cyclopropyl-2-(4-(methoxycarbonyl)phenyl)piperidin-1-yl)methyl)-5-(methoxy-d3 )-7-methyl-1H-indole-1-carboxylate (compound B)

Compound A (162 mg, 0.625 mmol) was dissolved in 5 mL of isopropyl acetate, maleic acid (73 mg, 0.628 mmol) was added, and the mixture was reacted at room temperature for 1 h. The reaction system was concentrated under reduced pressure to yield a crude product (235 mg); the crude product (235 mg) was dissolved in 10 mL of ethanol, tert-butyl 4-formyl-5-trideuteriomethoxy-7-methyl-1H-indole-1-carboxylate (165 mg, 0.564 mmol) was added, and 20 mg of Ir(CO)₂acac was added; and nitrogen replacement was performed three times, and the mixture was heated to 80°C and reacted under the atmosphere of hydrogen balloon for 16 h. The reaction solution was cooled to room temperature and concentrated under reduced pressure. The resulting crude product was separated and purified by silica gel column chromatography (petroleum ether : ethyl acetate (v/v) = 10 : 1) to afford compound B (200 g, yield: 60%).
LCMS m/z = 536.3 [M+1]⁺

### Step 2: Synthesis of compound 1

### 4-((2S,4R)-4-cyclopropyl-1-((5-(methoxy-d3)-7-methyl-1H-indol-4-yl)methyl)piperidin-2-yl)benzoic acid (compound 1)

Compound B (200 mg, 0.37 mmol) was dissolved in 5 mL of methanol, solid potassium carbonate (257 mg, 1.86 mmol) was added, and the mixture was heated to 80°C and reacted under reflux for 3 h. The reaction solution was cooled to room temperature and concentrated under reduced pressure to afford a crude product. The above-mentioned crude product was dissolved in a mixed solvent of 5 mL of THF and 1 mL of water, and lithium hydroxide monohydrate (155 mg, 3.7 mmol) was added and stirred at room temperature for 16 h. The reaction system was concentrated under reduced pressure, and the crude product was subjected to Pre-HPLC (instrument and preparative column: using Glison GX-281 preparative liquid chromatographic instrument, preparative column model: Sunfire C18, 5 µm, inner diameter × length = 30 mm×150 mm). Preparation method: the crude product was dissolved in methanol and dimethyl sulfoxide and filtered with a 0.45 µm filter membrane to prepare a sample liquid. Mobile phase system: acetonitrile/water (containing 0.1% TFA). Gradient elution method: gradient elution with acetonitrile from 5% to 60% (elution time: 15 min). Lyophilization was performed to afford a trifluoroacetate of **compound 1** (125 mg).
¹H NMR (400 MHz, CD₃OD) δ 8.28- 8.18 (m, 2H), 7.77- 7.68 (m, 2H), 7.36-7.30 (m, 1H), 6.77 (s, 1H), 6.32 (d, 1H), 4.48 (dd, 1H), 4.40- 4.29 (m, 1H), 4.18-4.08 (m, 1H), 3.62- 3.52 (m, 1H), 3.30- 3.21 (m, 1H), 2.51 (s, 3H), 2.25- 2.12 (m, 1H), 2.09 -1.85 (m, 2H), 1.79- 1.60 (m, 1H), 1.20- 1.03 (m, 1H), 0.67- 0.53 (m, 1H), 0.52- 0.40 (m, 2H), 0.26- 0.12 (m, 2H).
LCMS m/z = 422.2 [M+1]⁺

10 mL of 2-methyltetrahydrofuran and 50 mL of a saturated solution of sodium bicarbonate were added to 125 mg of the trifluoroacetate of **compound 1,** the mixture was stirred for 15 min and extracted, and the organic phase was concentrated to dryness to afford **compound 1** (60 mg).

### Example 2: preparation of crystalline form 1 of a succinic acid salt of compound 1

7.5 mL of ethanol and 2.5 mL of water were added to about 250 mg of **compound 1,** and the mixture was stirred at 25°C; 15 mL of a 47 mg/mL aqueous succinic acid solution was added, and the resulting mixture was stirred for 2 h and then subjected to suction filtration; and the filter cake was rinsed with a 30% aqueous ethanol solution and dried under vacuum to afford the crystalline form 1 of a succinic acid salt of **compound 1.**

### 1. Crystalline form test examples

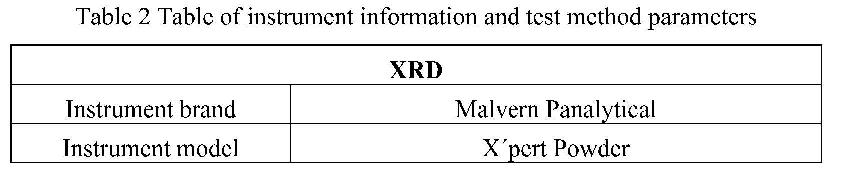

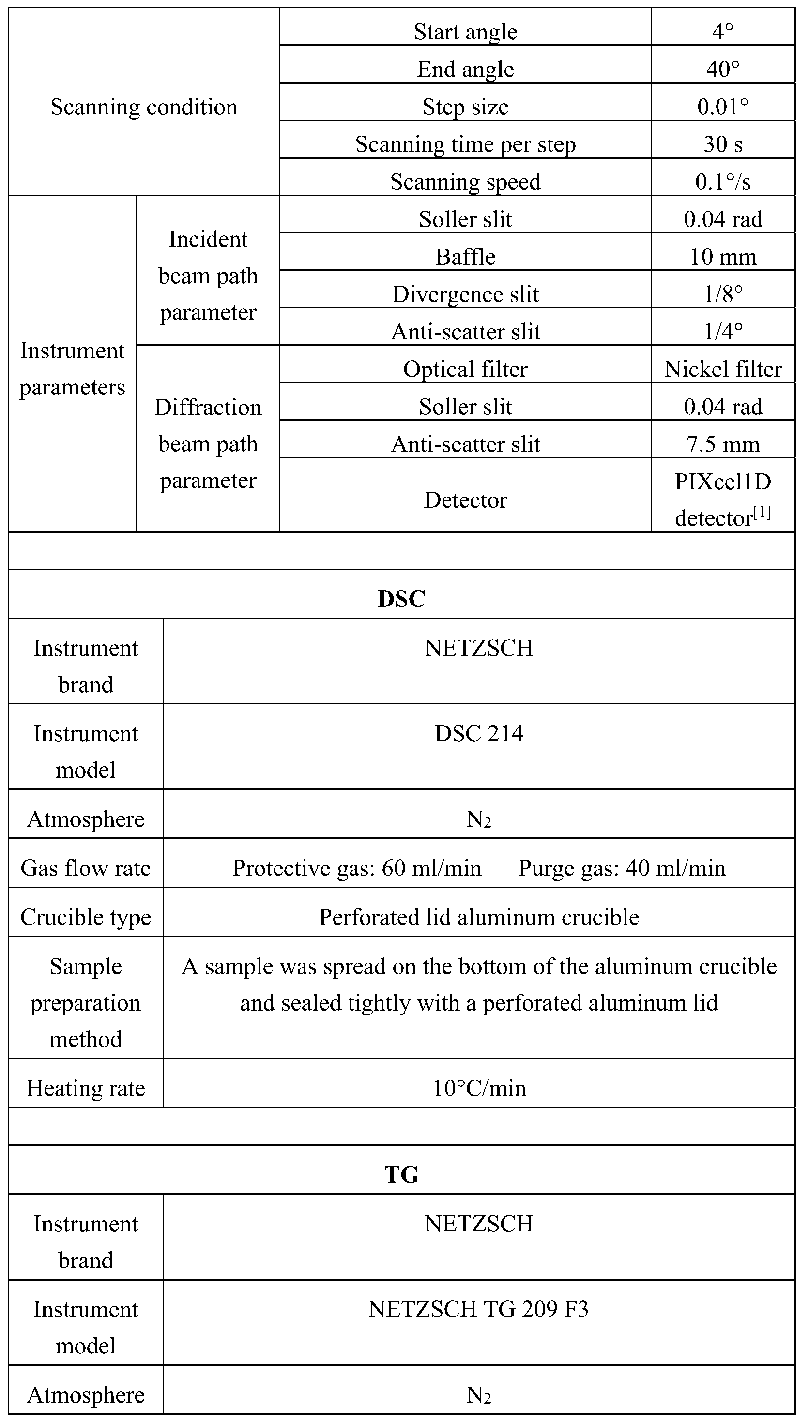

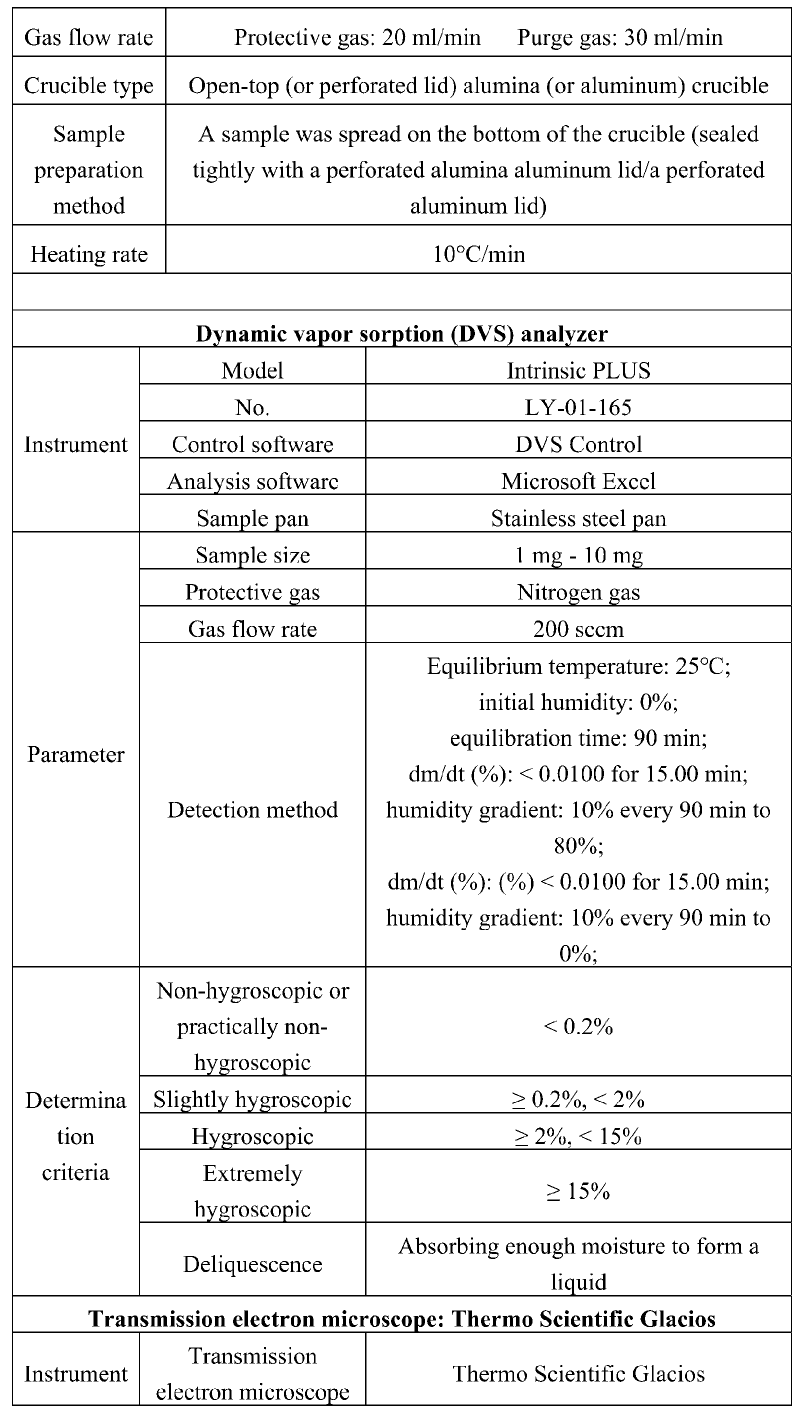

|

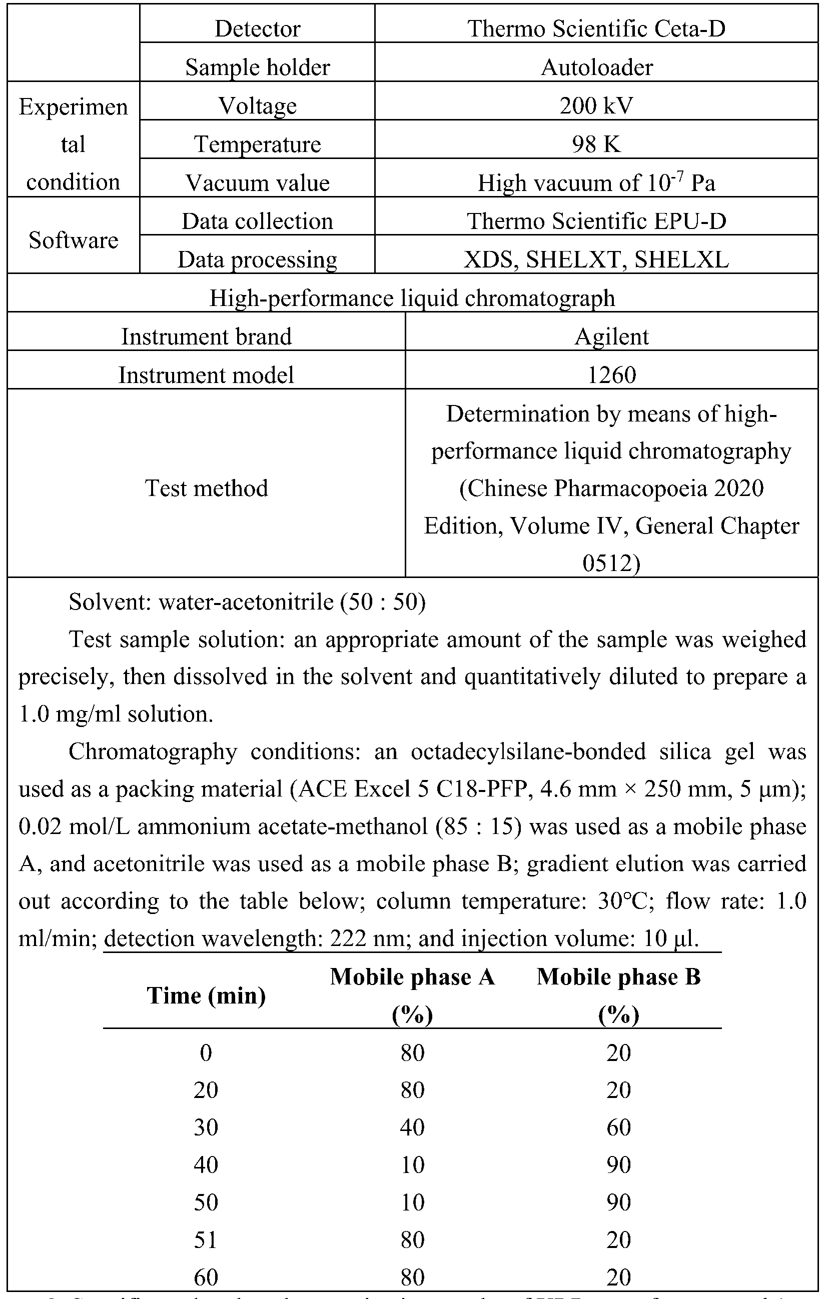

### 2. Specific peak value characterization results of XRD test of compound 1

The X-ray powder diffraction (XRD) pattern of the crystalline form of a succinic acid salt of compound I is as shown in Figure 1. Specific peak values are shown in Table 3.

**Table 3**

| Pos. [°2Th.] | Height [cts] | d-spacing | Rel. Int. [%] |
|---|---|---|---|
| 5.0743 | 1651.62 | 17.41539 | 15.16 |
| 7.3365 | 2913.6 | 12.04976 | 26.74 |
| 7.7676 | 10894.42 | 11.38201 | 100 |
| 8.1714 | 8691.72 | 10.82046 | 79.78 |
| 9.2721 | 3537.06 | 9.53827 | 32.47 |
| 10.1298 | 1128.17 | 8.73242 | 10.36 |
| 10.5586 | 2635.25 | 8.37875 | 24.19 |
| 10.854 | 2455.1 | 8.15142 | 22.54 |
| 12.4962 | 2842.54 | 7.08359 | 26.09 |
| 12.763 | 4710.93 | 6.93611 | 43.24 |
| 13.1888 | 1225.71 | 6.71313 | 11.25 |
| 13.6915 | 3230.01 | 6.46776 | 29.65 |
| 13.9174 | 2248.35 | 6.36327 | 20.64 |
| 14.6133 | 6142.33 | 6.0618 | 56.38 |
| 14.8631 | 2630.34 | 5.96048 | 24.14 |
| 15.1593 | 610.71 | 5.84467 | 5.61 |
| 15.738 | 1927.59 | 5.63104 | 17.69 |
| 16.3222 | 1508.12 | 5.43078 | 13.84 |
| 17.0636 | 6891.31 | 5.19646 | 63.26 |
| 17.3036 | 1510.57 | 5.12491 | 13.87 |
| 17.8493 | 973.29 | 4.96944 | 8.93 |
| 18.5497 | 812.72 | 4.78335 | 7.46 |
| 19.3757 | 1326.35 | 4.58126 | 12.17 |
| 19.759 | 2910.27 | 4.49326 | 26.71 |
| 19.9978 | 7782.7 | 4.44013 | 71.44 |
| 20.29 | 2216.55 | 4.37684 | 20.35 |
| 20.4753 | 1669.75 | 4.33764 | 15.33 |
| 20.8951 | 984.37 | 4.25143 | 9.04 |
| 21.0893 | 1192.92 | 4.21274 | 10.95 |
| 21.5225 | 3296.64 | 4.1289 | 30.26 |
| 21.762 | 1829.57 | 4.084 | 16.79 |
| 22.0131 | 2916.39 | 4.03798 | 26.77 |
| 22.4793 | 4393.87 | 3.95201 | 40.33 |
| 22.5643 | 3345.9 | 3.94709 | 30.71 |
| 23.0645 | 1010.93 | 3.85305 | 9.28 |
| 23.355 | 2118.35 | 3.80578 | 19.44 |
| 23.6304 | 1621.17 | 3.76204 | 14.88 |
| 24.1599 | 2056.11 | 3.68077 | 18.87 |
| 24.5339 | 2929.32 | 3.6255 | 26.89 |
| 25.5322 | 1861.85 | 3.48596 | 17.09 |
| 25.9353 | 1830.22 | 3.4327 | 16.8 |
| 26.5604 | 1588.07 | 3.35331 | 14.58 |
| 26.8726 | 613.8 | 3.31505 | 5.63 |
| 27.5094 | 1458.21 | 3.23974 | 13.38 |
| 28.0205 | 916.45 | 3.1818 | 8.41 |
| 28.3423 | 580.66 | 3.1464 | 5.33 |
| 28.6292 | 815.13 | 3.11552 | 7.48 |
| 29.0316 | 664.13 | 3.07325 | 6.1 |
| 29.2912 | 763.92 | 3.0466 | 7.01 |
| 30.264 | 413.9 | 2.95084 | 3.8 |
| 30.8783 | 342.76 | 2.89352 | 3.15 |
| 31.341 | 478.91 | 2.85185 | 4.4 |
| 31.7038 | 316.32 | 2.82004 | 2.9 |
| 32.246 | 232.99 | 2.77385 | 2.14 |
| 32.9304 | 291.97 | 2.71775 | 2.68 |
| 34.1942 | 277.34 | 2.62014 | 2.55 |
| 34.555 | 304.37 | 2.5936 | 2.79 |
| 35.4224 | 264.32 | 2.53205 | 2.43 |
| 36.8326 | 130.41 | 2.43828 | 1.2 |
| 38.1732 | 146.69 | 2.35567 | 1.35 |
| 38.6675 | 132.83 | 2.32669 | 1.22 |

### 3. Stability results

The stability results of the crystalline form 1 of a succinic acid salt of compound 1 are shown in Table 4.

**Table 4**

| Condition | Time | Purity |
|---|---|---|
| | 0 day | 97.80% |
| Accelerated: 40°C ± 2°C, 75% ± 5% RH | 1 month | 97.70% |
| | 2 months | 97.70% |
| | 3 months | 97.90% |
| | 6 months | 97.80% |
| Intermediate: 25°C ± 2°C, 60% ± 5% RH | 3 months | 98.00% |
| | 6 months | 97.80% |
| Long-term: 2°C - 8°C | 3 months | 97.90% |
| | 6 months | 97.70% |

Conclusion: the crystalline form 1 of a succinic acid salt of compound 1 showed good stability under accelerated, intermediate and long-term conditions.

### 4. Pharmacokinetic test in rats

**Experimental objective:** in this experiment, a single dose of the test substance was administered to SD rats intravenously and intragastrically, and the concentration of the test substance in plasma of the rats was measured to evaluate the pharmacokinetic characteristics of the test substance in the rats.

**Experimental animals:** male SD rats, 200-250 g, 6-8 weeks old, 6 rats/compound, purchased from Chengdu Dossy Experimental Animals Co., Ltd.

**Experimental method:** on the day of the experiment, 6 SD rats were randomly grouped according to their body weights. The rats were fasted with water available for 12-14 h one day before the administration, and were fed 4 h after the administration.

**Table 5**

| Group | Number | Administration information | | | | | | |
|---|---|---|---|---|---|---|---|---|
| | Male | Test substance | Administr ation dosage* (mg/kg) | Administr ation concentrat ion (mg/mL) | Administr ation volume (mL/kg) | Collected sample | Mode of administration | Vehicle |
| G1 | 3 | Compound of present invention | 10 | 1 | 10 | Plasma | Oral administration (Intragastric administration) | 0.5% MC |
| G2 | 3 | Compound of present invention | 1 | 0.2 | 5 | Plasma | Intravenous injection | 5% **DMA** + 5% Solutol + 90% Saline |

| | | | | | | | | |
|---|---|---|---|---|---|---|---|---|
| *Dosage is calculated based on a free base. | | | | | | | | |

**Sampling:** before and after the administration, 0.15 mL of blood was taken from the orbits under isoflurane anesthesia and placed in an EDTAK2 centrifuge tube. Centrifugation was carried out at 5000 rpm at 4°C for 10 min, and the plasma was collected.

The plasma collection time points for IV&PO groups were 0, 5 min, 15 min, 30 min, 1 h, 2 h, 4 h, 6 h, 8 h, and 24 h.

Before analysis and detection, all samples were stored at -60°C. The samples were analyzed quantitatively by LC-MS/MS.

**Table 6 Pharmacokinetic parameters of compound of present invention in plasma of rats Test compound**

| Test compound | Mode of administration* | Cmax (ng.mL-1) | T_{1/2} (h) | Tmax (h) | AUC₀₋₂₄ₕ (ng/mL·h) | F% |
|---|---|---|---|---|---|---|
| Trifluoroacetate of compound 1 | i.g. (10 mg/kg) | 1128 | 3.20 | - | 9164 | 72.1 |
| LNP023 | i.g. (10 mg/kg) | 867 | 3.39 | 3.5 | 9867 | 49 |

| | | | | | | |
|---|---|---|---|---|---|---|
| * Note: i.g. (intragastrical) administration of the compounds | | | | | | |

Conclusion: the compound of the present invention had good oral absorption in rats. Compared with LNP023, the trifluoroacetate of compound 1 had a higher maximum plasma concentration and higher oral bioavailability in rats.

Structure of the control compound (LNP023):

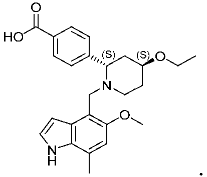

### 5. Pharmacokinetic test of dogs

**Experimental objective:** in this experiment, a single dose of the test substance was administered to beagle dogs intravenously and intragastrically, and the concentration of the test substance in plasma of the beagle dogs was measured to evaluate the pharmacokinetic characteristics of the test substance in the beagle dogs.

**Experimental animals:** male beagle dogs, 8-11 kg, 1-3 years old, 6 dogs/compound, purchased from Beijing Marshall Biotechnology Co., Ltd.

**Experimental method:** on the day of the experiment, 6 beagle dogs were randomly grouped according to their body weights. The beagle dogs were fasted with water available for 14-18 h one day before the administration, and were fed 4 h after the administration.

**Table 7**

| Group | Number | Administration information | | | | | | |
|---|---|---|---|---|---|---|---|---|
| | Male | Test substance | Administ ration dosage* (mg/kg) | Administrat ion concentrati on (mg/mL) | Administrat ion volume (mL/kg) | Collected sample | Mode of administr ation | Vehicle |
| G1 | 3 | Compoun d of present invention | *5* | 1 | *5* | Plasma | Oral administr ation (Intragast ric administr ation) | 0.5%MC (containing *0.5%* Tween 80) |
| G2 | 3 | Compoun d of present invention | 1 | 0.5 | 2 | Plasma | Intraveno us injection | 5% **DMA** + **5%** Solutol + 90% Saline |

| | | | | | | | | |
|---|---|---|---|---|---|---|---|---|
| *Dosage is calculated based on a free base. | | | | | | | | |

**Sampling:** before and after administration, 1.0 mL of blood was taken from the jugular veins or limb veins and placed in an EDTAK2 centrifuge tube. Centrifugation was carried out at 5000 rpm at 4°C for 10 min, and the plasma was collected.

The plasma collection time points for IV&PO groups were 0, 5 min, 15 min, 30 min, 1 h, 2 h, 4 h, 6 h, 8 h, 10 h, 12 h, and 24 h.

Before analysis and detection, all samples were stored at -60°C. The samples were analyzed quantitatively by LC-MS/MS.

**Table 8 Pharmacokinetic parameters of compound of present invention in plasma of dogs**

| Test compound | Mode of administration* | AUC₀₋ₜ (ng/mL·h) | T_{1/2} (h) | F% |
|---|---|---|---|---|
| Trifluoroacetate of compound 1 | i.g. (5 mg/kg) | 45447 | 15.1 | 75.5 |
| Trifluoroacetate of LNP023 | i.g. (4 mg/kg) | 19129 | 7.06 | 45.9 |

| | | | | |
|---|---|---|---|---|
| * Note: i.g. (intragastrical) administration of the compounds | | | | |

Conclusion: the compound of the present invention had good oral absorption in beagle dogs.

### 6. Pharmacokinetic test in monkeys

**Experimental objective:** in this experiment, a single dose of test substance was administered to cynomolgus monkeys intravenously and intragastrically, and the concentration of the test substance in plasma of the monkeys was measured to evaluate the pharmacokinetic characteristics of the test substance in the monkeys.

**Experimental animals:** male cynomolgus monkeys, 3-5 kg, 3-6 years old, 6 monkeys/compound, purchased from Suzhou Xishan Biotechnology Co., Ltd.

**Experimental method:** on the day of the experiment, 6 monkeys were randomly grouped according to their body weights. The cynomolgus monkeys were fasted with water available for 14-18 h one day before the administration, and were fed 4 h after the administration.

**Table 9**

| Group | Number | Administration information | | | | | | |
|---|---|---|---|---|---|---|---|---|
| | Male | Test substance | Administ ration dosage* (mg/kg) | Administ ration concentra tion (mg/mL) | Administrat ion volume (mL/kg) | Collected sample | Mode of administr ation | Vehicle |
| G1 | 3 | Compound of present invention | *5* | 1 | *5* | Plasma | Oral administr ation (Intragast ric administr ation) | 0.5%MC (containing *0.5%* Tween 80) |
| G2 | 3 | Compound of present invention | 1 | 0.5 | 2 | Plasma | Intraveno us injection | 5% DMA + 5% Solutol + 90% Saline |

| | | | | | | | | |
|---|---|---|---|---|---|---|---|---|
| *Dosage is calculated based on a free base. | | | | | | | | |

**Sampling:** before and after the administration, 1.0 mL of blood was taken from the limb veins and placed in an EDTAK2 centrifuge tube. Centrifugation was carried out at 5000 rpm at 4°C for 10 min, and the plasma was collected.

The plasma collection time points for IV&PO groups were 0, 5 min, 15 min, 30 min, 1 h, 2 h, 4 h, 6 h, 8 h, 10 h, 12 h, and 24 h.

Before analysis and detection, all samples were stored at -60°C. The samples were analyzed quantitatively by LC-MS/MS.

**Table 10 Pharmacokinetic parameters of compound of present invention in plasma of monkeys**

| Test compound | Mode of administration* | AUC₀₋ₜ (ng/mL·h) | F% |
|---|---|---|---|
| Trifluoroacetate of | i.g. (5 mg/kg) | 48279 ± 3664 | 98.3 ± 7.5 |
| compound 1 | | | |
| Trifluoroacetate of LNP023 | i.g. (5 mg/kg) | 13188 ± 1472 | 35.2 ± 3.9 |

| | | | |
|---|---|---|---|
| * Note: i.g. (intragastrical) administration of the compounds | | | |

Conclusion: the compound of the present invention had good oral absorption in monkeys. Compared with the trifluoroacetate of LNP023, the trifluoroacetate of compound 1 had higher oral exposure and bioavailability in monkeys.

### 7. Test of Caco2 permeability

In the experiment, a monolayer of Caco-2 cells was used, and incubated in triplicate in a 96-well Transwell plate. A transport buffer solution (HBSS, 10 mM HEPES, pH 7.4 ± 0.05) containing the compound of the present invention (2 µM) or the control compound digoxin (10 µM), nadolol (2 µM) or metoprolol (2 µM) was added to the administration end hole on the apical side or basal side. A DMSO-containing transport buffer solution was added to the corresponding receiving end hole. After incubation for 2 hours at 37°C ± 1°C, the cell plate was removed, and an appropriate amount of samples was taken from both the apical side and basal side and transferred to a new 96-well plate. Subsequently, acetonitrile containing an internal standard was added to precipitate the protein. The samples were analyzed using LC MS/MS, and the concentrations of the compound of the present invention and the control compound were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was evaluated by leakage of Lucifer Yellow.

**Table 11 Caco2 test results of compound of present invention**

| Test compound | Mean Pₐₚₚ (10⁻⁶ cm/s) | | Efflux Ratio |
|---|---|---|---|
| | A to B | B to A | |
| Trifluoroacetate of compound 1 | 1.77 | 19.4 | 11.0 |
| Trifluoroacetate of LNP023 | 0.570 | 14.4 | 25.3 |

Conclusion: the compound of the present invention had good Caco2 permeability. Compared with the trifluoroacetate of LNP023, the trifluoroacetate of compound 1 had better permeability and a lower efflux ratio.

## Claims

1. A pharmaceutically acceptable salt of a compound represented by formula (I), 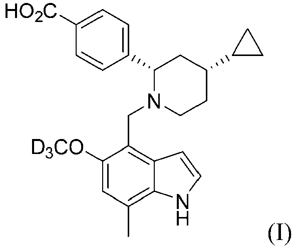 **characterized in that** the pharmaceutically acceptable salt is selected from succinic acid salts, with the molar ratio of the compound represented by formula (I) to succinic acid being about 1 : 0.75.

2. A crystalline form 1 of a succinic acid salt of a compound represented by formula (I), **characterized in that** the crystalline form 1 belongs to the orthorhombic crystal system with a space group C222₁ (No. 20), with lattice constants being a = 12.66(3) Å, b = 23.79(3) Å, c = 34.40(6) Å, α = 90°, β = 90° and γ = 90°, a unit cell volume V = 10364(36) Å3, and the Z' of the system being 2.

3. A crystalline form 1 of a succinic acid salt of a compound represented by formula (I), **characterized in that** the crystalline form 1 has an X-ray powder diffraction pattern with characteristic diffraction peaks at the following 20 positions (20 ± 0.2°), determined using Cu-Kα radiation: 7.77° ± 0.2°, 8.17° ± 0.2°, 14.61° ± 0.2°, 17.06° ± 0.2°, and 20.00° ± 0.2°.

4. The crystalline form 1 of the succinic acid salt of the compound represented by formula (I) according to claim 2, **characterized in that** the crystalline form 1 has an X-ray powder diffraction pattern with characteristic diffraction peaks at the following 20 positions (2θ ± 0.2°), determined using Cu-Kα radiation: 7.77° ± 0.2°, 8.17° ± 0.2°, 9.27° ± 0.2°, 12.76° ± 0.2°, 14.61° ± 0.2°, 17.06° ± 0.2°, 20.00° ± 0.2°, and 22.48° ± 0.2°.

5. The crystalline form 1 of the succinic acid salt of the compound represented by formula (I) according to claim 2, **characterized in that** the crystalline form 1 has an X-ray powder diffraction pattern with characteristic diffraction peaks at the following 20 positions (2θ ± 0.2°), determined using Cu-Kα radiation: 7.34° ± 0.2°, 7.77° ± 0.2°, 8.17° ± 0.2°, 9.27° ± 0.2°, 12.50° ± 0.2°, 12.76° ± 0.2°, 13.69° ± 0.2°, 14.61° ± 0.2°, 17.06° ± 0.2°, 19.76° ± 0.2°, 20.00° ± 0.2°, 21.52° ± 0.2°, 22.01° ± 0.2°, 22.48° ± 0.2°, 22.56° ± 0.2°, and 24.53° ± 0.2°.

6. The crystalline form 1 of the succinic acid salt according to claim 2, **characterized in that** the crystalline form 1 has an X-ray powder diffraction pattern as shown in Figure 1, determined using Cu-Kα radiation.

7. The crystalline form 1 of the succinic acid salt according to claim 2, **characterized in that** the crystalline form 1 has a differential scanning calorimetry curve and a thermogravimetric analysis curve as shown in Figure 2 and Figure 3, respectively.

8. A method for preparing a crystalline form 1 of a succinic acid salt of a compound represented by formula (I), **characterized in that** the method comprises: (1) adding a solvent (i) to a compound represented by formula (I), and stirring same at room temperature or under heating; and (2) adding an aqueous succinic acid solution, and stirring and crystallizing same, followed by drying to obtain the target crystalline form 1.

9. The preparation method according to claim 8, **characterized in that** the solvent used is selected from one of a C₁₋₆ halogenated alkane solvent, a C₂₋₆ ester solvent, a C₂₋₆ nitrile solvent, a C₂₋₆ ether solvent, a C₁₋₆ alcohol solvent or water or a mixed solvent of two or more thereof at any ratio, preferably one or more of dichloromethane, 1,2-dichloroethane, acetonitrile, ethyl acetate, methanol, ethanol, isopropanol, propanol, diethyl ether, tetrahydrofuran or water.

10. A pharmaceutical composition, **characterized in that** the pharmaceutical composition comprises a therapeutically effective amount of the pharmaceutically acceptable salt according to claim 1 or the crystalline form according to any one of claims 2-7, and a pharmaceutically acceptable carrier or excipient.

11. The pharmaceutical composition according to claim 10, **characterized in that** the therapeutically effective amount is 1-800 mg calculated based on a free base.

12. Use of the pharmaceutically acceptable salt according to claim 1, or the crystalline form according to claims 2-7, or the pharmaceutical composition according to claim 10 in the preparation of a drug for treating a disease related to the activity or expression level of complement factor B.

13. The use according to claim 12, **characterized in that** the disease is selected from kidney diseases.
